(19) Europäisches Patentamt
European Patent Office
Office européen des brevets

(11) Publication number: **0 104 041**
**B1**

(12) # EUROPEAN PATENT SPECIFICATION

(45) Date of publication of patent specification: **27.07.88**

(51) Int. Cl.$^4$: **C 07 K 7/02,** C 12 Q 1/36, A 61 K 37/02

(21) Application number: **83305353.1**

(22) Date of filing: **13.09.83**

(54) **Enzyme inhibitors.**

(30) Priority: **15.09.82 GB 8226273**
**01.02.83 US 462928**

(43) Date of publication of application:
**28.03.84 Bulletin 84/13**

(45) Publication of the grant of the patent:
**27.07.88 Bulletin 88/30**

(84) Designated Contracting States:
**DE FR GB IT SE**

(56) References cited:
**EP-A-0 045 665**
**EP-A-0 077 028**
**EP-A-0 081 783**

(73) Proprietor: **Aktiebolaget Hässle**
**Kärragatan 5**
**S-431 83 Mölndal (SE)**

(72) Inventor: **Szelke, Michael**
**10 North Drive**
**Ruislip, Middlesex (GB)**
Inventor: **Jones, David Michael**
**89 Woodrow Avenue**
**Hayes, Middlesex (GB)**
Inventor: **Hallet, Allen**
**60 Kingsdom Road**
**Cheam, Surrey (GB)**
Inventor: **Atrash, Butrus**
**9 Testwood Court, Golden Manor**
**Hanwell London W.7 (GB)**

(74) Representative: **Näsman, Rune B. G. et al**
**AB Astra Patent and Trade Mark Department**
**S-151 85 Södertälje (SE)**

**EP 0 104 041 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to renin-inhibiting peptide analogues.

Background

Renin is a natural enzyme, disorders in relation to which are implicated in many cases of hypertension. It is released into the blood from the kidney, and cleaves from a blood glycoprotein a decapeptide known as angiotensin-I. Circulating angiotensin-I is cleaved in lung, kidney and other tissues to an octapeptide, angiotensin-II, which raises blood pressure both directly by causing arteriolar constriction and indirectly by stimulating release of the sodium-retaining hormone aldosterone from the adrenal gland and thus causing a rise in extracellular fluid volume. The latter effect is caused by angiotensin-II itself or a heptapeptide cleavage product antiotensin-III.

Inhibitors of renin have therefore been sought, with two ends in view, first the provision of a diagnostic agent for identification of cases of hypertension due to renin excess, and secondly the provision of an agent for control of hypertension in such cases.

The present inventors' approach has been to consider the peptide sequence characterising the natural renin substrate at its binding site, and to seek peptide analogues sufficiently similar to bind to the enzyme, in competition with the natural substrate, but sufficiently dissimilar to it to be cleaved slowly or not at all. Such analogues will block the action of the enzyme and attack the hypertension at source.

Renin is specific to a particular bond in the substrate, the N-terminal sequence of which in the horse is for example:

C ↓ (Asp–Arg), B ↓ (Phe–His), A ↓ (Leu–Leu)

Asp–Arg–Val–Tyr–Ile–His–Pro–Phe–His–Leu–Leu–Val–Tyr–Ser– (IA)

1 2 3 4 5 6 7 8 9 10 11 12 13 14

as found by L. T. Skeggs et al J. Exper. Med. *106* 439 (1957). Human renin substrate has a different sequence recently discovered by D. A. Tewkesbury et al Biochem. Biophys. Res. Comm. *99* 1311 (1981)

A ↓

–Val–Ile–His– (IB)

11 12 13

the sequence to the left of the arrow A being as in formula (IA).

Cleavage at A gives angiotensin-I; subsequent cleavage at the Phe-His bond at B gives angiotensin-II; and cleavage subsequently again at the Asp-Arg bond at C gives angiotensin-III.

Peptides similar to certain partial sequences of the substrate have been shown to act as inhibitors of renin in vitro. An example is the tetrapeptide ester (the relation to the substrate residues being indicated by numbering):

H–Leu–Leu–Val–Phe–OMe (II)

10 11 12 13

proposed by Kokubu, Nature, *217* 456 (1968) but it is inactive in vivo, because of binding to plasma proteins and rapid attack by natural peptidases.

One of the present inventors undertook some years ago a development of Kokubu's work, seeking a renin inhibitor active in vivo, in which analogues of peptides similar to Kokubu's were made but having a methylene imino group —$CH_2$—NH— in place of the peptide link —CO—NH— between the leucine residues. One of these analogues was:

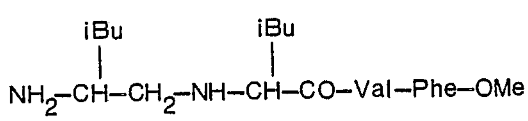 (III)

10 11 12 13

which is the tetrapeptide (I) modified at the Leu-Leu link, leucine of course being

$$NH_2\text{—}\overset{\overset{\text{iBu}}{|}}{CH}\text{—}COOH \qquad (IV)$$

This analogue (III) was the first effective in-vivo inhibitor of renin and was shown to have significant antihypertensive action in Goldblatt hypertensive rats (Parry, Russell and Szelke p. 541 in "Chemistry and Biology of Peptides" Ed. Meienhofer, Ann Arbor Science Publishers 1972). Little or no attention has however been paid to the work, which the authors themselves were unable to pursue, in spite of considerable activity in the general field of substrate-based inhibitors for renin, reviewed for example by Haber & Burton, Federation Proc. *38* No. 13 2768—2773 (1979).

The Invention

The present invention is a development of the above work, to which the inventors were stimulated by consideration of the acid protease inhibitor pepstatin further considered later herein. It contains the acid statine $NH_2CH(iBu)CH(OH)CH_2COOH$, an amino acid (though not an alpha amino acid as the common natural amino acids are) and appeared to the inventors to offer scope for work related to though on different lines from their work on peptide analogues disclosed for example in their U.S. patent application Serial No. 290 620 filed 5 August 1981 (published as European Patent Specification No. 0 045 665 on 10th February 1982).

In those analogues a peptide bond is represented by other links corresponding to partial or complete reduction at the carbonyl group and/or replacement of the nitrogen (—NH—) group by a methylene group. The inventors have however now surprisingly found that notional replacement of the carbonyl group by a number of 2-, 3- or 4-carbon groups including but by no means restricted to the group seen in statine gives renin-inhibiting peptide analogues of high specificity and activity. This is so even though the structural relation to the polypeptide that is the natural substrate of renin is less than previously. Low activity would be expected and, further, loss of specificity as for example with pepstatin itself.

Thus behind both inventions is a concept of modified peptide structures related to the peptide sequence at the site of action of renin on the natural substrate, by substitution at the site of cleavage, but the two approaches are distinct.

Optionally further in the present invention there is isosteric substitution, or other modification, at other positions to increase stability or to modify the properties of the final peptide, for example its solubility under physiological conditions or its resistance to degradation in vivo. Such modification may for example be by incorporation of residues other than those of the natural L-amino acids; by protection of the N-terminus with acetyl, pivaloyl, *t*-butyloxycarbonyl (Boc), benzoyl or other groups; or by conversion of the C-terminal carboxyl to another functional group, e.g. the corresponding alcohol, present as such or in ether or ester form.

THE PRESENT COMPOUNDS DEFINED

General reference to amino acids and amino acyl residues and side chains in both the description and claims wherein is to be taken as reference to such whether naturally occurring in proteins or not and to both D- and L- forms, and amino is to be taken as including imino.

The compounds of the pressent invention, showing desirable renin inhibitory action, are of the general formula:

X-Y-Pro-Phe-His—A—B—His-W (V)

6 7 8 9 10,11 12 13

or the partial sequences:

X-A-B-His-W (V) (i)

X-His-A-B-His-W (V) (ii)

X-Phe-His-A-B-His-W (V)(iii)

X-Pro-Phe-His-A-B-His-W (V) (iv)

where:

Pro, Phe, and His may be in substituted form, e.g. carrying OH, F, Cl, Br or Me;

X = H; or an acyl or other N-protecting group e.g. acetyl, pivaloyl, benzyloxycarbonyl, *t*-butyloxycarbonyl (Boc), benzoyl or lower alkyl (primarily $C_1$—$C_5$); or an D- or L- amino acyl residue (especially Pro), which may itself be N-protected similarly:

Y = D- or L-His or other D- or L- basic or aromatic amino-acyl residue, or is absent;

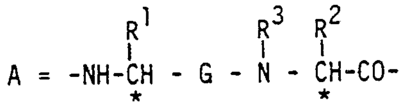 (VI) where

G = 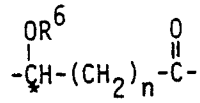 (VII) with n = 0, 1, 2, 3

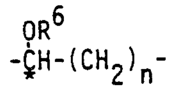 (VIII) n = 1, 2, 3, 4

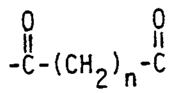 (IX) n = 0, 1, 2, 3

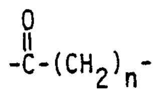 (X) n = 1, 2, 3, 4

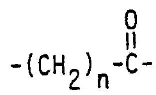 (XI) n = 1, 2, 3

$-(CH_2)_n-$ (XII) n = 3, 4

and where the configuration at asymmetric centres* is either R or S, $R^1$ and $R^2$, the same or different, being = $^i$Pr (isopropyl), $^i$Bu (isobutyl), Bzl (benzyl) or other amino-acid side chain preferably lipophilic or aromatic;

$R^3$ = H; lower alkyl ($C_1$—$C_5$); or t-butyloxycarbonyl, benzyloxycarbonyl, ring substituted benzyloxycarbonyl, —$SO_2PH$, —$SO_3C_6H_4CH_3$(p), formyl or other N-protecting group including lower acyl $C_1$—$C_5$) generally;

$R^6$ = H, or lower alkyl, lower acyl, benzyl, tetrahydropyranyl, or other hydroxyl protecting group;

B = D- or L- Val Leu or Ile or other D- or L- lipophilic amino-acyl residue; and

W = i) —OH as such as in protected ester form e.g. as —$OR^4$ where $R^4$ = lower alkyl primarily $C_1$—$C_5$ and particularly $^t$Bu, or cycloalkyl primarily $C_3$—$C_7$, or Bzl, or other ester forming group; or ii) —$NH_2$, as such, or in protected amide form as —$NHR^5$ or —$N(R^5)_2$ (where $R^5$ = an N-protecting or other substituent group e.g. lower alkyl as for $R^4$ and $(R^5)_2$ = two such groups or e.g. cyclo-alkyl, primarily $C_3$—$C_7$), or as —NH—$(CH_2)_n$—Q or —$NR^5$—$(CH_2)_n$—Q (where n = 2 to 6 and Q = $NH_2$ or

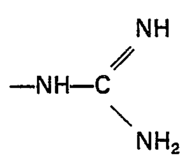

and wherein any of the hydrogens attached to nitrogen may be substituted by $R^5$ or $(R^5)_2$); or iii) an D- or L- serine or D- or L- lysine, arginine or other basic amino-acyl residue as such or in amide form, substituted amide form or ester form e.g. containing a group or groups as given for $R^4$ and $R^5$ above as the case may be; or iv) an amino alcohol residue derived therefrom as such or protected in ester or ether form e.g. containing a group as given for $R^4$ above or

His + W = the alcohol L- or D-His as such or protected in ester or ether form as above;

such polypeptide being in the above form or modified by isosteric replacement of one or more remaining peptide bonds, for example by reduced —$CH_2$—NH—, keto,

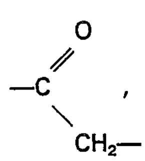 ,

hydroxy, —CH(OH)—$CH_2$—, or hydrocarbon —$CH_2$—$CH_2$— isosteric links in the form:

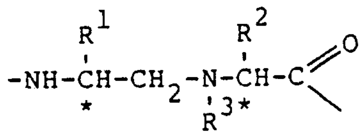

(XIII) "reduced" isostere bond

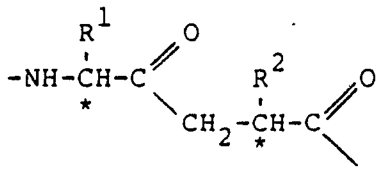

(XIV) "keto" isostere bond

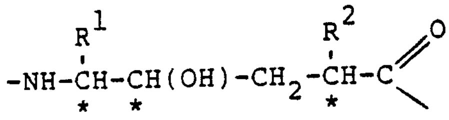

(XV) "hydroxy" isostere bond

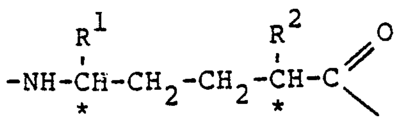

(XVI) "hydrocarbon" isostere bond

where the significance of *, $R^1$, $R^2$ and $R^3$ is as before;
and said polypeptide further being in free form or in protected form at one or more remaining amino or amide (including peptide) nitrogen, carboxyl, hydroxy or other reactive groups, or in salt form at amino, imidazole or carboxyl groups, in particular as thir physiologically acceptable acid addition salts at basic centres.

The above compounds may in particular be those related to the substrate sequence in the horse (B = Val at position 12) or those related to the substrate sequence in man (B = Ile at position 12). Particular groups of these compounds are set out in the formulae below, either:

(A) X–Y–Pro–Phe–His——A——Val–His–W (VA)
6 7 8 9 10,11 12 13

where
X, Y, Pro, Phe and His are as before
A is as before except that
$R^1$ and $R^2$, the same or different = $^{i}$Bu (isobutyl), $^{i}$Pr or Bzl (benzyl) or other amino-acid side chain preferably lipophilic or aromatic
$R^3$ = —H; or —$SO_2Ph$, —$SO_2C_6H_4CH_3$(p), Boc, formyl or other N-protecting group
$R^6$ = lower alkyl, lower acyl, benzyl, tetrahydropyranyl or other hydroxyl protecting group
W = —OH as such or in protected ester form as —$OR^4$ where
$R^4$ = lower alkyl (primarily $C_1$—$C_5$ and particularly $^{t}$Bu), or Bzl, or other ester forming group; or —$NH_2$ as such or in protected amide form as —$NHR^5$ or —$N(R^5)_2$ ($R^5$ = an N-protecting group e.g. lower alkyl as for $R^4$; $(R^5)_2$ = two such or e.g. cyclo-alkyl, primarily $C_3$—$C_7$) or an L- or D- amino-acyl residue e.g. a serine or basic amino-acyl residue as such or in amide form or in protected amide or ester form e.g. containing a group or groups as given for $R^4$ and $R^5$ above as the case maybe; or an amino acid alcohol residue derived therefrom as such or protected in ester or ether form e.g. containing a group as given for $R^4$ above or
His + W = the alcohol derived from L- or D- His as such or protected in ester or ether form as above; or:

(B) X–Y–Pro–Phe–His——A——Ile–His–W (VB)
6 7 8 9 10,11 12 13

where
X, Y, Pro, Phe and His are as before
A is as before except that
$R^1$ = $^{i}$Bu (isobutyl) or Bzl (benzyl) or other amino-acid side chain preferably lipophilic or aromatic
$R^2$ = $^{i}$Pr (isopropyl), and
$R^3$ = —H; or —$SO_2Ph$, —$SO_2C_6H_4CH_3$(p), Boc, formyl or other N-protecting group
$R^6$ = lower alkyl, lower acyl, benzyl, tetrahydropyranyl, or other hydroxyl protecting group
W is as in formula (VA) or
His + W = the alcohol derived from His, as such or protected in ester or ether form.

The numbering of residues in formulae (V), (VA) and (VB) shows the correspondence with the renin

substrates themselves, but without limitation of the generality of the formulae.

Substitutes for Pro, Phe and His above may for example be: (*1*) for Pro 4-hydroxyproline (HPro) or pGlu (*2*) for Phe: Tyr, Phe(4-Cl), Phe(4-F) (*3*) for His: His(Me), Spinacin.

Reference to basic and aromatic amino acids above, and to amino acids with lipophilic side chains includes but is not restricted to the common amino acids of those classes, viz:

| | | | |
|---|---|---|---|
| Basic: | Arginine<br>Lysine<br>Histidine | | |
| Aromatic: | Phenylalanine<br>Tyrosine<br>Tryptophan<br>Histidine | | |
| Lipophilic: | Leucine<br>Isoleucine<br>Valine | Phenylalanine<br>Cyclohexylalanine<br>Adamantylalanine | } unnatural |

Suitable amino acyl residues X may for example be those of: D or L Pro, Val or Ile; Gly.

Where a peptide bond in addition to that corresponding to the Leu-Leu or Leu-Val bond in the natural renin substrate is modified, the 7, 8 and 8, 9 positions i.e. the Pro-Phe and Phe-His bonds in formula V are preferred, or possibly both of these positions, and it is further preferred that the substitution should be

$$-CH_2-\underset{\displaystyle R^3}{\underset{|}{N}}- \qquad \text{(XVII)}$$

i.e. as a "reduced" isostere bond XIII, where $R^3$ is as set out above. The alternative isosteric substitutions set out herein may however be used.

Protective or substituent groupings as mentioned above may be any of these known in the polypeptide art, amply disclosed in the literature and not requiring discussion at length here. Generally the selection of the groups is according to their function, some being primarily intended to protect against undesired reaction during synthetic procedures while the N- and C- terminal substituents are for example directed against the attack of enzymes on the final compounds or to increase their solubility and hence physiological acceptability. All these functions are generally within the terms "protective group" or the like used herein, including the claims. It is in particular possible for one or more remaining peptide bonds in the compounds of formula (V), VA) or (VB) to be N-substituted.

Statine

A particular representative of the group

$$-NH-\overset{\displaystyle R^1}{\overset{|}{CH}}-G- \qquad \text{(XVIII)}$$

above is the S,S-statine residue (Sta)

$$-NH-\underset{*}{\overset{\displaystyle iBu}{\overset{|}{CH}}}-\underset{*}{\overset{\displaystyle OH}{\overset{|}{CH}}}-CH_2-CO- \qquad \text{(XIX)}$$

Statine itself is a known compound occurring in nature in the protease inhibitor pepstatin isolated in 1970 by Japanese workers from various Actinomycetes (Morishima et al J. Antibiot. *13* No. 5 259—265 (1970)) of formula:

iso-valeryl-L-valyl-L-valyl-S,S-statyl-L-alanyl-S,S-statine (XX)

or, in the usual notation:

iso-valeryl-Val-Val-Sta-Ala-Sta-OH (XXI)

it inhibits acid proteases in general, so called because the catalytic functional groups are carboxyl groups of aspartic acid residues (as opposed to serine residues in serine proteases). Pepsin, cathepsin D, chymosin and renin are some of the representatives of this class of enzymes, and all are inhibited by pepstatin.

Incorporated however for example in the compound

H-His-Pro-Phe-His-Sta-Val-Ile-His-OH (H 176) (XXII)

statine when tested by the methods in our published European specification gives $IC_{50} = 0.016\ \mu M$ against human renin. This compound is the subject of Example 1 below.

Applications

The invention further lies

i) In a diagnostic test for high renin states, blood pressure falling most when renin is high, and a surgical prognostic test for reno-vascular hypertension (renal artery stenosis), by the administration of a polypeptide analogue as above followed by monitoring of blood pressure, and such polypeptide analogues when for such use, and

ii) In the long and short term treatment of heart failure and all forms of hypertension particularly those associated with high serum renin levels, by the administration of a renin-inhibiting amount of a polypeptide analogue as above, and such polypeptide analogues when for such use.

The long and short term response of blood pressure to renin inhibitors is predictive of surgical outcome. In all cases single and repeated doses and any conventional form of pharmaceutical composition may be used, for administration by intranasal or oral route, injection, or any other means as convenient. Amounts may for example be 0.001 to 10 mg/kg body weight daily more usually 0.01 to 1 mg, according to the potency of the analogue and the severity of the condition. Dosage unit compositions may contain such amounts or submultiples thereof to make up the daily dose.

(Dosages herein are related to the free base content where compounds are in salt form.)

Synthetic Methods

The inventors have developed synthetic methods for the isosteric replacement of the peptide bond —CONH— with alternative groups, specifically $—CH_2—NH—$ (reduced), $—CH_2CH_2$ (hydrocarbon),

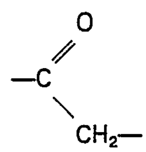

(keto) and $—CH(OH)—CH_2—$ (hydroxy) isosteres as referred to earlier herein, see e.g. Szelke, et al, pp. 57—70 in "Molecular Endocrinology" Vol. *1,* Editors: MacIntyre and Szelke, Elsevier, Amsterdam 1977; Hudson, Sharpe and Szelke, U.S. Patent 4 198 398 "Enkephalin Analogues"; and Szelke, Jones and Hallett (Ferring Pharmaceuticals Ltd. and Ferring AB) in the published European specification already referred to.

Reference may be made to these publications for general discussion of such isosteric replacement and, in the European specification, for discussion in relation to renin inhibitors particularly. Reaction sequences for the preparation of peptide analogues applicable in the context of the present invention, apart from the link at A, are given there.

Turning therefore to the link at A, first there are several known syntheses of statine (XXIII below) available, e.g. that by D. H. Rich et al, J. Org. Chem. 1978, 43, p.3624 and references quoted therein. Deoxystatine (XXIV below) has also been described (D. H. Rich et al, BBRC 1977, *74,* p762), as has the synthesis of nor-statine (XXV below) and its various analogues (R. Nishizawa et al, J. Med. Chem. 1977, *20,* p.510).

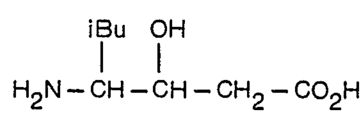

(XXIII)

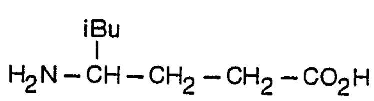

(XXIV)

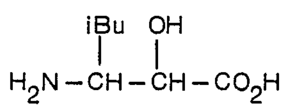

(XXV)

Of these, comparing with the possibilities for G given earlier, XXIII has

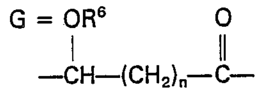

where $R^6$ = H and n = 1;
XXIV has

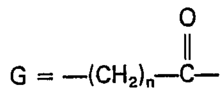

where n = 2; and
XXV has

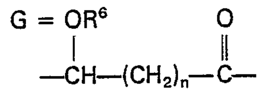

where $R^6$ = H and n = 0.

For example, the following specific compounds have been made:

H-His-Pro-Phe-His-Sta-Val-Ile-His-OH (H—176) (XXII)

H-Pro-His-Pro-Phe-His-Sta-Val-Ile-His-Lys-OH (H—189) (XXXVI)

Their preparation was carried out by the procedures generally as described in our published European specification, and given in detail in the Examples which appear below. Statine was incorporated in the form of Nα-Boc-Statine, using DCCI and HOBt as coupling reagents.

Other structures for G of particular value are:

1 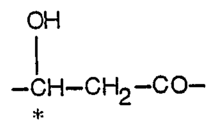 (XXVI)

2 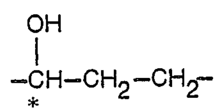 (XXVII)

3 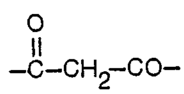 (XXVIII)

4 $-CH_2-CH_2-CO-$ (XXIX)

5 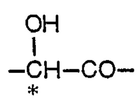 (XXX)

6 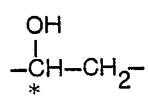 (XXXI)

7 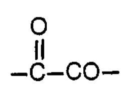 (XXXII)

8 $-CH_2-CO-$ (XXXIII)

9 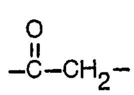 (XXXIV)

Syntheses of these, generally applicable but given by way of example in the context of the detailed synthesis of Example 1 and thus giving octapeptide analogues of H—176 containing the various structures, are respectively as follows, referring to the reaction schemes given below:

*1* By coupling of Boc-Sta-OH to the appropriate protected peptide, which in the synthesis for example of H—176 is H-Val-Ile-His(Bom)—O® (LVI) (® = resin support)

2 By coupling compound L (Scheme II below, R = H) to H-Ile-His-(Bom)—O® (LVII)

3 By oxidising Boc-statine with pyridinium dichromate and coupling the resultant keto acid

$$\text{Boc-NH—CHR}^1\text{—CO—CH}_2\text{—CO}_2\text{H} \qquad \text{(XXXVII)}$$

($R^1$ = $^i$Bu) to the tripeptide ester LVI above

4 By coupling Boc-deoxy-statine

$$\text{Boc-NH—CHR}^1\text{—CH}_2\text{—CH}_2\text{—CO}_2\text{H} \qquad \text{(XXXVIII)}$$

($R^1$ = $^i$Bu) prepared according to Rich et al, BBRC 1977, *74,* p 762, to the tripeptide ester LVI.

5 By coupling Boc-*nor*-statine (obtained according to R. Nishizawa et al, J. Med. Chem. 1977, *20,* p. 510) to the tripeptide ester LVI.

6 By coupling compound XLVI (Scheme I below, R = H) to the dipeptide ester LVII above

7 By oxidising Boc-*nor*-statine to the corresponding keto acid

$$\text{Boc-NR—CHR}^1\text{—CO—CO}_2\text{H} \qquad \text{(XXXIX)}$$

and coupling the latter to the tripeptide ester LVI.

8 By coupling Boc-NH—$CHR^1$—$CH_2$—$CO_2H$ obtained by one cycle of the Arndt-Eistert reaction from Boc-NH—$CHR^1$—$CO_2H$,) to the tripeptide ester LVI.

9 By oxidising the hydroxyl group in compound XLVI (Scheme I, R = H) to a keto group and coupling the resultant dipeptide analogue to the dipeptide ester LVII.

The same methods are applicable in making compounds corresponding to that of Example 2.

Scheme I

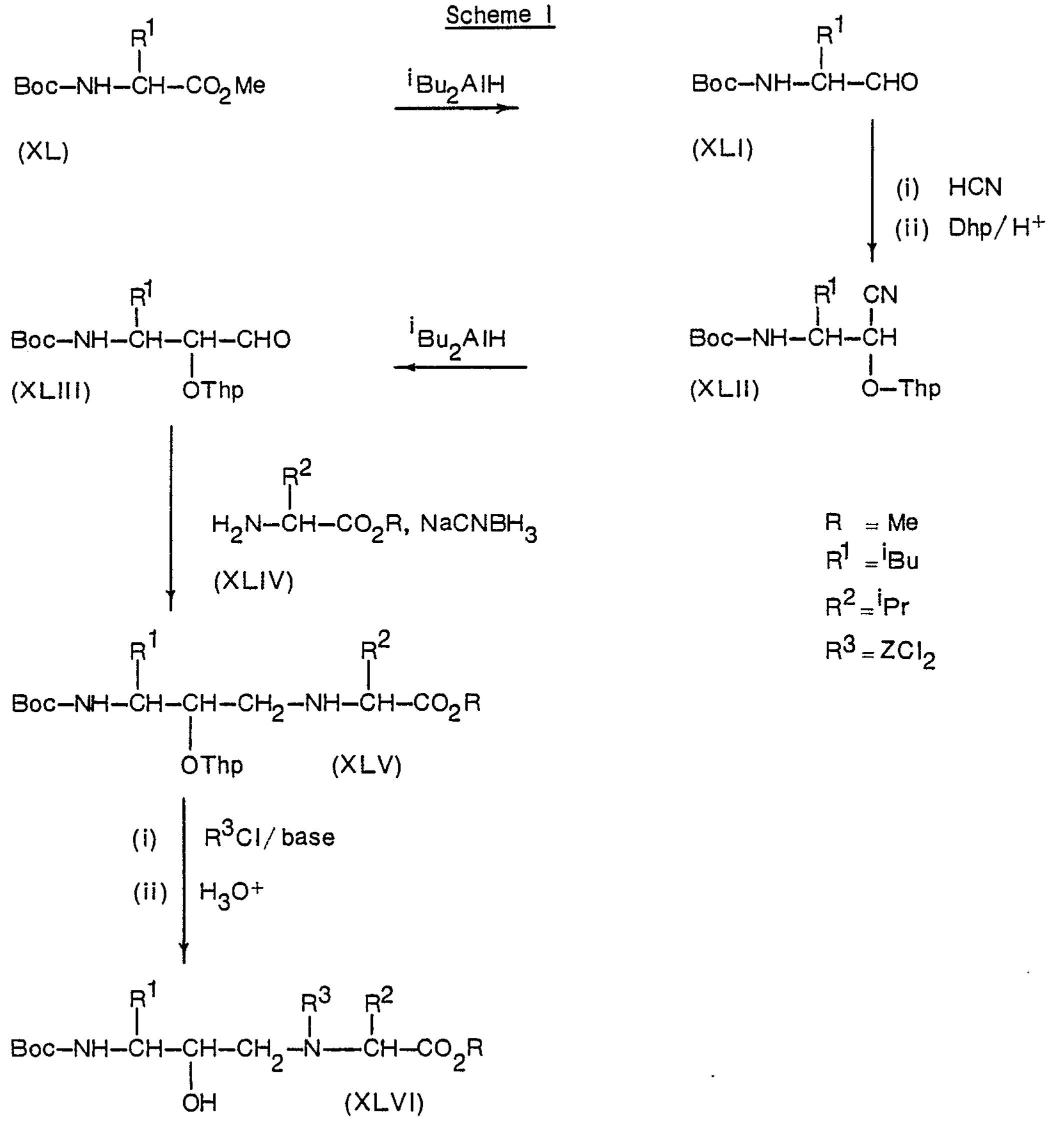

Scheme II

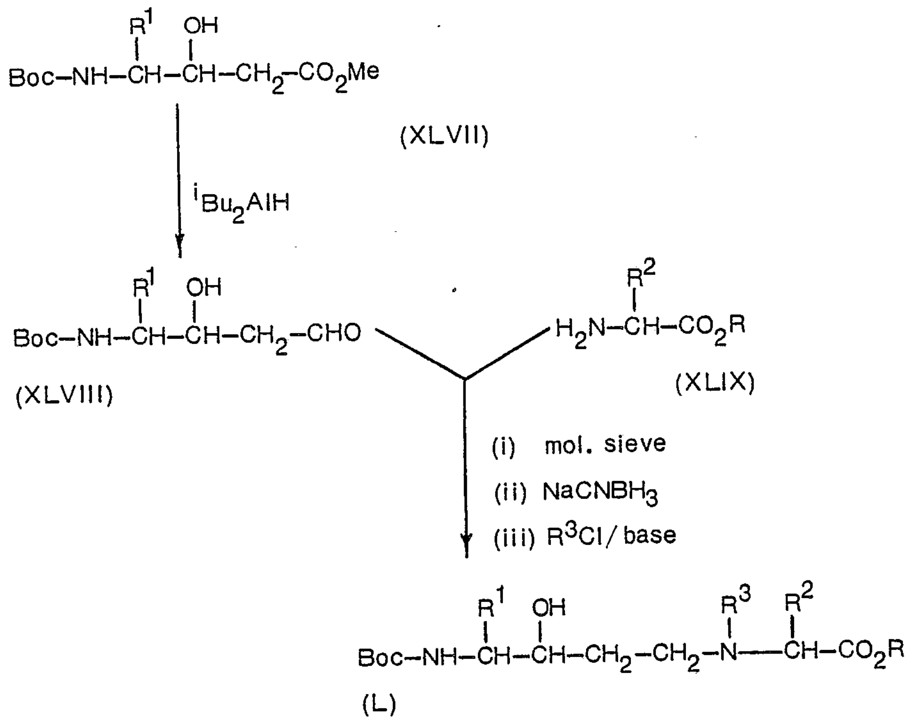


(XLVII)

(XLVIII)

(XLIX)

(L)

R = Me or Bzl
$R^1$ = $^iBu$
$R^2$ = $^iPr$
$R^3$ = $ZCl_2$

In the above syntheses *1* to *9* and Schemes I and II:

| | | |
|---|---|---|
| Boc | = | tert-butoxy carbonyl |
| DCCI | = | N, N'-dicyclohexyl-carbodi-imide |
| HOBt | = | 1-hydroxybenzotriazole |
| Bom | = | π-benzyloxymethyl |
| Dhp | = | 2,3-dihydro-4H-pyranyl |
| Thp | = | 2-tetrahydropyranyl |
| $ZCl_2$ | = | 3,4-dichlorobenzyloxycarbonyl |

## EXAMPLES

The following are the fully detailed examples referred to earlier. The activity of H—176 (Example I) has already been given. Preliminary results for H—189 (Example II) indicate still higher activity, $IC_{50}$ 0.009 μM against human renin and 0.012 μM against baboon renin.

### Example 1

H—176 *H-His-Pro-Phe-His-Sta-Val-Ile-His-OH* (XXII)

Boc-His (π-Bom)-O-Resin (1 g, 0.2 mmol) was washed with reagents as described in European Application 0 045 665 Example I. Subsequent deprotections and coupling reactions were carried out by using the same sequence of washes and reaction times with the following modifications.

During the coupling of Boc-Sta-OH to H-Val-Ile-His-(π-Bom)-O-Resin, the reaction was allowed to proceed for 16 hrs and 0.27 mmoles of Boc-statine were used. For all the other coupling reactions, 0.8 mmoles of Boc-amino acids were used.

After the final coupling, followed by acetylation for 1 hr, the resin was washed and dried to give 0.926 g of product.

This material was treated with HF at 0° for 1¼ hrs. in the presence of anisole (1 ml) and dried overnight over potassium hydroxide. The resin was washed with acetic acid/water (1:1) (100 ml) to remove the peptide. Evaporation of volatiles afforded a residue which upon drying over KOH under high vacuum weighed 126 mgs.

This material was applied to a Sephadex G.25 column (82 × 2.5 cm), eluted with 50% acetic acid at 19.2 ml/hr, collecting 6.4 ml fractions. Fractions 27—39 were pooled, evaporated and the residue dried over KOH under high vacuum (67.5 mg). This residue was applied to a CM—52 ion-exchange column (30 × 1 cm) and eluted with an ammonium acetate gradient 0.05 M to 0.5 M over 2 days at 5.8 ml/hr collecting 2.9 ml fractions. After collecting 50 fractions, no peak assignable to the product was obtained on the trace. Therefore, the column was eluted with 0.5 M ammonium acetate taking 5.8 ml fractions. The product from fraction 55 was found to be pure. Lyophilisation afforded 7.4 mgs of pure material.

H—176 $C_{60}H_{82}O_{10}N_{14}$ MW: 1099.356

*TLC:* Rf = 0.4 in EtoAc/Py/AcoH/$H_2O$ (20:20:6:11) on silica plates

Amino acid analysis: after hydrolysis in 6N HCl plus phenol for 92 hrs, 110°, peptide content 88.6% His: 2.97; Pro: 1.1, Val: 0.98; Ile: 0.97; Phe: 0.99

Example 2

H—189, *H-Pro-His-Pro-Phe-His-Sta-Val-Ile-His-Lys-OH* (XXXVI)

Solid-phase synthesis of this peptide was carried out by the described procedure starting with 1.2 grams (0.24 mmole) of Boc-Lys ($Cl_2Z$)-O-Resin. In the subsequent coupling reactions, 1 mmole of Boc-amino acid was used except for Boc-Statine of which 0.36 mmoles were used. The dried peptide-Resin ester weighed 1.53 grams.

0.6 grams of this material were subjected to HF + anisole treatment and afforded a residue of 171.6 mgs. This residue was applied to a G—25 Sephadex column (82 × 2.5 cm) eluted with 50% acetic acid at 19.2 ml/hr collecting 6.4 ml fractions. Fractions 32—42 provided 121.6 mgs of residue. Half of this material was applied to a CM—52 ion-exchange column (30 × 1 cm), eluted with an ammonium acetate buffer gradient from 0.05 M to 1 M over 2 days at 5.8 ml/hr collecting 2.9 ml fractions. Fraction 33 afforded pure product which upon lyophilisation gave 9.5 mgs of material.

H—189 $C_{66}H_{101}O_{12}N_{17}$ MW: 1204.535

*TLC* (silica) Rf = 0.5 in EtoAc/Py/AcoH/$H_2O$ (15:20:6:11)

Amino acid analysis after hydrolysis with 6N HCl + phenol at 110°/40 hrs:
His: 3.03; Ile: 0.90; Phe: 0.86; Pro: 2.19; Val: 0.94. Peptide content 80.1%.

Use of the Compounds

Either of the above compounds may be given, as described earlier herein, in amounts of for example 0.1 mg/kg body weight daily in diagnosis of high-renin states and in therapy of heart failure and hypertension.

**Claims**

1. Polypeptide analogues of the general formula:

X-Y-Pro-Phe-His-A-B-His-W (V)

or the partial sequences

X-A-B-His-W (V) (i)

X-His-A-B-His-W (V) (ii)

X-Phe-His-A-B-His-W (V) (iii)

X-Pro-Phe-His-A-B-His-W (V) (iv)

where

X = hydrogen, an amino-nitrogen protecting group or an amino acyl residue;

Y = a D- or L- basic or aromatic amino-acyl residue;

$$A = -NH-\underset{*}{\overset{R^1}{CH}} - G - \overset{R^3}{N} - \underset{*}{\overset{R^2}{CH}}-CO- \qquad (VI) \quad \text{where}$$

$$G = -\underset{*}{\overset{OR^6}{C}}H-(CH_2)_n-\overset{O}{\overset{\|}{C}}- \qquad (VII) \quad \text{with } n = 0, 1, 2, 3$$

$$-\underset{*}{\overset{OR^6}{C}}H-(CH_2)_n- \qquad (VIII) \quad n = 1, 2, 3, 4$$

$$-\overset{O}{\overset{\|}{C}}-(CH_2)_n-\overset{O}{\overset{\|}{C}} \qquad (IX) \quad n = 0, 1, 2, 3$$

$$-\overset{O}{\overset{\|}{C}}-(CH_2)_n- \qquad (X) \quad n = 1, 2, 3, 4$$

$$-(CH_2)_n-\overset{O}{\overset{\|}{C}}- \qquad (XI) \quad n = 1, 2, 3$$

$$-(CH_2)_n- \qquad (XII) \quad n = 3, 4$$

and where the configuration at asymmetric centres ★ is either R or S and $R^1$ and $R^2$, the same or different, are amino acid side chains;

$R^3$ = hydrogen or an amino-nitrogen protecting group;

$R^6$ = hydrogen or a hydroxyl protecting group;

B = a lipophilic amino acyl residue; and

W = a hydroxyl group, an amide nitrogen group, or a basic amino acyl residue or amino alcohol residue derived therefrom; or

His + W = the amino alcohol residue corresponding to His.

2. Polypeptide analogues according to claim 1, where

$$G = -\underset{*}{\overset{OR^6}{C}}H-CH_2-\overset{O}{\overset{\|}{C}}- \qquad (VII)$$

$$-\underset{*}{\overset{OR^6}{C}}H-(CH_2)_2- \qquad (VIII)$$

$$-\overset{O}{\overset{\|}{C}}-CH_2-\overset{O}{\overset{\|}{C}}- \qquad (IX)$$

$$-\overset{O}{\overset{\|}{C}}-(CH_2)_2- \qquad (X)$$

$$-(CH_2)_2\ -\overset{O}{\overset{\|}{C}}- \qquad (XI)$$

$$-(CH_2)_3- \qquad (XII)$$

3. A polypeptide analogue according to claim 1, wherein one or more of said Pro Phe and His are substituted in the ring, Pro by OH, Phe by OH, F, Cl, Br or Me, His by Me, or are replaced by, respectively, 4-hydroxyproline (HPro) or pGlu; Tyr, Phe(4-Cl) or Phe(4-F); His (Me) or spinacin.

4. A polypeptide analogue according to claim 1 or 2 wherein said protecting group X is an acyl or lower

alkyl group and particularly said acyl group is acetyl, pivaloyl, *t*-butyoxycarbonyl (Boc) benzyloxycarbonyl or benzoyl and said lower alkyl group is a $C_1$—$C_5$ alkyl group.

5. A polypeptide analogue according to claim 1 or 2, wherein said amino acyl group X is D- or L- Pro, Val or Ile, or Gly, and/or is in protected form carrying a group as set out in claim 3.

6. A polypeptide analogue according to any preceding claim, wherein said amino acyl residue Y is D- or L-His.

7. A polypeptide analogue according to any preceding claim, wherein said amino acid side chains $R^1$ and $R^2$ are lipophilic or aromatic, e.g. isopropyl, isobutyl and benzyl, and particularly those of Leu Ile Val Phe cyclohexyl-Ala adamantyl-Ala, or Phe Tyr Trp or His.

8. A polypeptide analogue according to any preceding claim, wherein said protecting group $R^3$ is lower alkyl, lower acyl, —$SO_2Ph$, —$SO_2C_6H_4CH_3$(p), *t*-butoxy carbonyl (Boc), benzyloxycarbonyl or ring substituted benzyloxycarbonyl, and particularly wherein said alkyl or acyl group is a $C_1$—$C_5$ group.

9. A polypeptide analogue according to any preceding claim, wherein said hydroxyl protecting group $R^6$ is lower alkyl or lower acyl, particularly $C_1$—$C_5$ alkyl or acyl, or benzyl or tetrahydropyranyl.

10. A polypeptide analogue according to any preceding claim, wherein said residue B is a D- or L-Val Leu or Ile residue.

11. A polypeptide analogue according to any preceding claim, wherein said hydroxyl group W is protected in ester form, e.g. by a lower alkyl or cycloalkyl group, or Bzl, and particularly wherein said lower alkyl group is a $C_1$—$C_5$ alkyl group and said cycloalkyl group a $C_3$—$C_7$ cycloalkyl group.

12. A polypeptide analogue according to any preceding claim, wherein said amide nitrogen group W is in protected form, especially

i) a group —$NHR^5$ or —$N(R^5)_2$ where $R^5$ is lower alkyl and $(R^5)_2$ is two lower alkyl groups the same or different or a cycloalkyl group, particularly where the or each said alkyl group is a $C_1$—$C_8$ alkyl group and said cycloalkyl group is a $C_3$—$C_8$ cycloalkyl group or

ii) a group —NH—$(CH_2)_n$—Q or $NR^5$—$(CH_2)_n$—Q where n = 2 to 6 and Q = an amino or guanidyl group where $R^5$ is lower alkyl, particularly where one or more of the hydrogens attached to nitrogen in said protecting group is replaced by $R^5$ or $(R^5)_2$, defined as above.

13. A polypeptide analogue according to any preceding claim, wherein said amino acid residue W is a D- or L- Ser, Lys or Arg residue as such or as

i) an amide or protected amide particularly when protected by a group as set out in claim 12, or

ii) an ester particularly when the esterifying group is as set out in claim 11.

14. A polypeptide analogue according to any preceding claim, wherein said amino alcohol residue W is the amino alcohol residue derived from D- or L- Ser, Lys or Arg or said amino alcohol residue Z + W is derived from D- or L- Tyr Phe or His, in either case as such or protected in ester or ether form, e.g. by a protective group $R^6$ as set out in claim 8.

15. A polypeptide analogue according to any preceding claim, wherein G is selected from:

1. 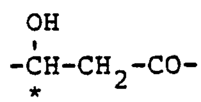 (XXVI)

2. 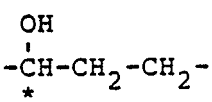 (XXVII)

3. 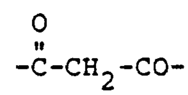 (XXVIII)

4. $-CH_2-CH_2-CO-$ (XXIX)

16. A polypeptide analogue according to any preceding claim, wherein said polypeptide analogue is in further modified form by isosteric replacement of one or more remaining peptide bonds by

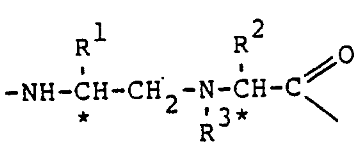 (XIII) "reduced" isostere bond

or

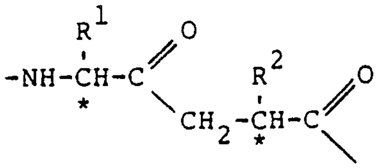

(XIV) "keto" isostere bond

or

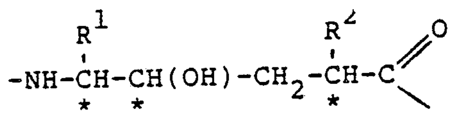

(XV) "hydroxy" isostere bond

or

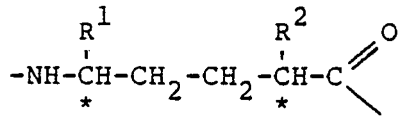

(XVI) "hydrocarbon" isostere bond

where the significance of ★, $R^1$, $R^2$ and $R^3$ is as before, particularly where one or both of the Pro-Phe and Phe-His bonds in formula V is the site of the isosteric replacement.

17. A polypeptide analogue according to any preceding claim, wherein said polypeptide analogue is in protected form at one or more remaining amino or amide (including peptide) nitrogen, carboxyl, hydroxy or other reactive groups, or in salt form at amino, imidazole or carboxyl groups, or is as an acid addition salt at one or more basic centres.

18. Polypeptide analogue of the general formula:

X-Y-Pro-Phe-His—A—B—His-W (V)
6 7 8 9 10,11 12 13

or the partial sequences

X-A-B-His-W (V) (i)

X-His-A-B-His-W (V) (ii)

X-Phe-His-A-B-His-W (V) (iii)

X-Pro-Phe-His-A-B-His-W (V) (iv)

where:

Pro, Phe, and His may be in substituted form, e.g. carrying OH, F, Cl, Br or Me;

X = H; or an acyl or other N-protecting group e.g. acetyl, pivaloyl,benzyloxycarbonyl, *t*-butyloxycarbonyl (Boc), benzoyl or lower alkyl (primarily $C_1$—$C_5$); or an D- or L- amino acetyl residue (especially Pro), which may itself be N-protected similarly:

Y = D- or L-His or other D- or L- basic or aromatic amino-acyl residue, or is absent;

A = 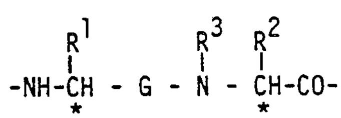 (VI) where

G = 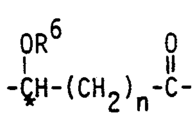 (VII) with n = 0, 1, 2, 3

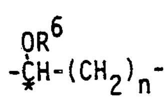 (VIII) n = 1, 2, 3, 4

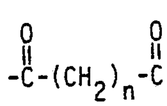 (IX) n = 0, 1, 2, 3

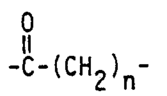 (X) n = 1, 2, 3, 4

$$-(CH_2)_n-\overset{O}{\overset{\|}{C}}- \qquad (XI) \qquad n = 1, 2, 3$$

$$-(CH_2)_n- \qquad (XII) \qquad n = 3, 4$$

and where the configuration at asymmetric centres ★ is either R or S, $R^1$ and $R^2$, the same or different, being = $^iPr$ (isopropyl), $^iBu$ (isobutyl), Bzl (benzyl) or other amino-acid side chain preferably lipophilic or aromatic;

$R^3$ = H; lower alkyl ($C_1$—$C_5$); or t-butyloxycarbonyl, benzyloxycarbonyl, ring substituted benzyloxycarbonyl, —$SO_2PH$, —$SO_2C_6H_4CH_3$(p), formyl or other N-protecting group including lower acyl ($C_1$—$C_5$) generally;

$R^6$ = H, or lower alkyl, lower acyl, benzyl, tetrahydropyranyl, or other hydroxyl protecting group;

B = D- or L- Val Leu or Ile or other D- or L- lipophilic amino-acyl residue; and

W =

i) —OH as such or in protected ester form e.g. as —$OR^4$ where $R^4$ = lower alkyl primarily $C_1$—$C_5$ and particularly $^tBu$, or cycloalkyl primarily $C_3$—$C_7$, or Bzl, or other ester forming group; or

ii) —$NH_2$, as such, or in protected amide form as —$NHR^5$ or —$N(R^5)_2$ (where $R^5$ = an N-protecting or other substituent group e.g. lower alkyl as for $R^4$ and $(R^5)_2$ = two such groups or e.g. cyclo-alkyl, primarily $C_3$—$C_7$), or as —NH—$(CH_2)_n$—Q or —$NR^5$—$(CH_2)_n$—Q (where n = 2 to 6 and Q = $NH_2$ or

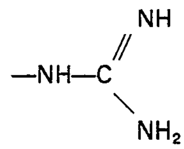

and wherein any of the hydrogens attached to nitrogen may be substituted by $R^5$ or $(R^5)_2$); or

iii) an D- or L- serine or D- or L- lysine, arginine or other basic amino-acyl residue as such or in amide form, substituted amide form or ester form e.g. containing a group or groups as given for $R^4$ and $R^5$ above as the case may be; or

iv) an amino alcohol residue derived therefrom as such or protected in ester or ether form e.g. containing a group as given for $R^4$ above or His + W = an alcohol derived from L- or D-His.

such polypeptide being in the above form or modified by isosteric replacement of one or more remaining peptide bonds, for example by reduced —$CH_2$—NH—, keto,

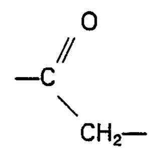

hydroxy, —CH(OH)—$CH_2$—, or hydrocarbon —$CH_2$—$CH_2$— isosteric links in the form:

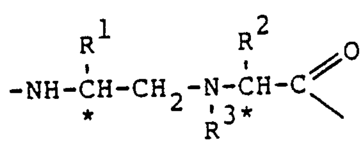 (XIII) "reduced" isostere bond

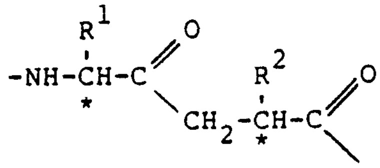 (XIV) "keto" isostere bond

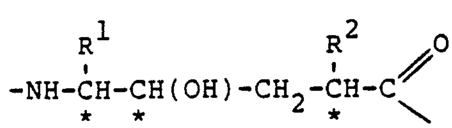 (XV) "hydroxy" isostere bond

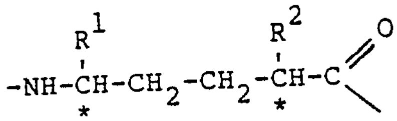 (XVI) "hydrocarbon" isostere bond

where the significance of ★, $R^1$, $R^2$ and $R^3$ is as before; and said polypeptide further being in free form or in protected form at one or more remaining amino or amide (including peptide) nitrogen, carboxyl, hydroxy or other reactive groups, or in salt form at amino, imidazole or carboxyl groups, in particular as their physiologically acceptable acid addition salts at basic centres.

19. The polypeptide analogues:

H-His-Pro-Phe-His-Sta-Val-Ile-His-OH

H-Pro-His-Pro-Phe-His-Sta-Val-Ile-His-Lys-OH

or corresponding analogues wherein the —$CH(OH)CH_2CO$— link of the statine residue is replaced by one of the links set out in claim 15.

20. A polypeptide analogue according to any preceding claim for use in a diagnostic test for high renin states wherein a polypeptide analogue is administered, a positive result being indicated by a fall in blood pressure related to said administration, or for use in treatment of heart failure or hypertension wherein an effective amount of a polypeptide analogue is administered.

21. A pharmaceutical composition comprising a polypeptide analogue according to any preceding claim 1 to 19, in a pharmaceutically acceptable medium.

**Patentansprüche**

1. Polypeptidanaloges der allgemeinen Formel:

X-Y-Pro-Phe-His-A-B-His-W (V)

oder die Teilsequenzen

X-A-B-His-W (V) (i)

X-His-A-B-His-W (V) (ii)

X-Phe-His-A-B-His-W (V) (iii)

X-Pro-Phe-His-A-B-His-W (V) (iv)

worin

X = Wasserstoff, eine Aminostickstoff-Schutzgruppe oder ein Aminoacylrest;

Y = ein basischer oder aromatischer D- oder L-Aminoacylrest;

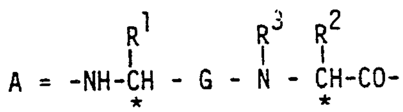 (VI) worin

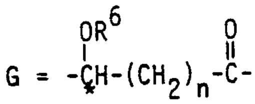 (VII) mit n = 0, 1, 2, 3

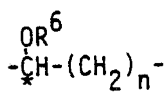 (VIII) n = 1, 2, 3, 4

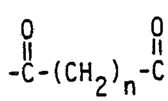 (IX) n = 0, 1, 2, 3

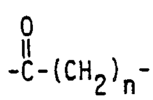 (X) n = 1, 2, 3, 4

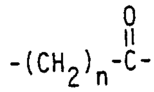 (XI) n = 1, 2, 3

$-(CH_2)_n-$ (XII) n = 3, 4

und worin die Konfiguration an asymmetrischen Zentren ★ entweder R oder S ist und $R^1$ und $R^2$, die gleich oder verschieden sind, Aminosäureseitenketten sind;

$R^3$ = Wasserstoff oder eine Aminostickstoff-Schutzgruppe;

$R^6$ = Wasserstoff oder eine Hydroxyl-Schutzgruppe;

B = ein lipophiler Aminoacylrest; und

W = eine Hydroxylgruppe, eine Amidstickstoffgruppe oder ein basischer Aminoacylrest oder ein sich davon ableitender Aminoalkoholrest ist; oder

His + W = der His entsprechende Aminoalkoholrest.

2. Polypeptidanaloges nach Anspruch 1, worin

G = 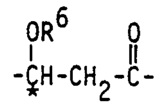 (VII)

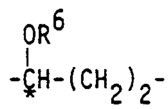 (VIII)

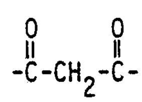 (IX)

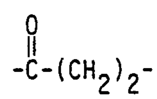 (X)

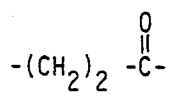 (XI)

$-(CH_2)_3-$ (XII)

3. Polypeptidanaloges nach Anspruch 1, worin eines oder mehrere von Pro, Phe und His im Ring substituiert sind, Pro durch OH, Phe durch OH, F, Cl, Br oder Me, His durch Me, oder jeweils durch 4-Hydroxyprolin (HPro) oder pGlu; durch Tyr, Phe(4-Cl) oder Phe(4-F) bzw. His (Me) oder Spinacin ausgetauscht sind.

4. Polypeptidanaloges nach Anspruch 1 oder 2, bei dem die Schutzgruppe X eine Acyl- oder niedermolekulare Alkylgruppe ist und insbesondere die Acylgruppe Acetyl, Pivalolyl, tert-Butoxycarbonyl (Boc), Benyloxycarbonyl oder Benzoyl ist und die niedermolekulare Alkylgruppe eine $C_1$—$C_5$-Alkylgruppe ist.

5. Polypeptidanaloges nach Anspruch 1 oder 2, bei dem die Aminoacylgruppe X D- oder L- Pro, Val oder Ile oder Gly ist und/oder in geschützter Form vorliegt und eine in Anspruch 3 genannte Gruppe trägt.

6. Polypeptidanaloges nach einem der vorausgehenden Ansprüche, worin der Aminoacylrest Y D- oder L- His ist.

7. Polypeptidanaloges nach einem der vorausgehenden Ansprüche, bei dem die Aminosäureseitenketten $R^1$ und $R^2$ lipophil oder aromatisch sind, z.B. Isopropyl, Isobutyl und Benzyl, und besonders jene von Leu Ile Val Phe Cyclohexyl-Ala Adamantyl-Ala oder Phe Pyr Trp oder His.

8. Polypeptidanaloges nach einem der vorausgehenden Ansprüche, worin die Schutzgruppe $R^3$ niedermolekulares Alkyl, niedermolekulares Acyl, $—SO_2Ph$, $—SO_2C_6H_4CH_3(p)$, tert-Butoxycarbonyl (Boc), Benzyloxycarbonyl oder ringsubstituiertes Benzyloxycarbonyl ist und worin insbesondere die Alkyl- oder Acylgruppe eine $C_1$—$C_5$-Gruppe ist.

9. Polypeptidanaloges nach einem der vorausgehenden Ansprüche, bei dem die Hydroxyl-Schutzgruppe $R^6$ niedermolekulares Alkyl oder niedermolekulares Acyl, besonders $C_1$—$C_5$-Alkyl oder -Acyl oder Benzyl oder Tetrahydropyranyl ist.

10. Polypeptidanaloges nach einem der vorausgehenden Ansprüche, bei dem der Rest B ein D- oder L-Val, -Leu- oder -Ile-Rest ist.

11. Polypeptidanaloges nach einem der vorausgehenden Ansprüche, bei dem die Hydroxylgruppe W in Esterform geschützt ist, z.B. durch eine niedermolekulare Alkyl- oder Cycloalkylgruppe oder Bzl, und worin insbesondere die niedermolekulare Alkylgruppe eine $C_1$—$C_5$-Alkylgruppe und die Cycloalkylgruppe eine $C_3$—$C_7$-Cycloalkylgruppe ist.

12. Polypeptidanaloges nach einem der vorausgehenden Ansprüche, bei dem die Amidstickstoffgruppe W in geschützter Form vorliegt, insbesondere

i) als Gruppe —$NHR^5$ oder —$N(R^5)_2$, worin $R^5$ eine niedermolekulare Alkylgruppe ist und $(R^5)_2$ zwei gleiche oder verschiedene niedermolekulare Alkylgruppen oder eine Cycloalkylgruppe bedeutet, wobei insbesondere die oder jede Alkylgruppe eine $C_1$—$C_5$-Alkylgruppe ist und die Cycloalkylgruppe eine $C_3$—$C_7$-Cycloalkylgruppe ist, oder

ii) als Gruppe —NH—$(CH_2)_n$-Q oder $NR^5$—$(CH_2)_n$-Q, worin n = 2 bis 6 und Q = eine Amino- oder Guanidylgruppe, worin $R^5$ niedermolekulares Alkyl ist, wobei insbesondere ein oder mehrere der an Stickstoff gebundenen Wasserstoffatome in der Schutzgruppe durch wie oben definiertes $R^5$ oder $(R^5)_2$ ersetzt sind.

13. Polypeptidanaloges nach einem der vorausgehenden Ansprüche, worin der Aminosäurerest W ein D- oder L-Ser-, -Lys- oder -Arg-Rest als solcher oder als

i) ein Amid oder geschütztes Amid, insbesondere durch eine wie in Anspruch 12 definierte Gruppe geschützt, oder

ii) als ein Ester, insbesondere durch die wei in Anspruch 11 definierte veresternde Gruppe geschützt, ist.

14. Polypeptidanaloges nach einem der vorausgehenden Ansprüche, bei dem der Aminoalkoholrest W der Aminoalkoholrest ist, der sich von D- oder L-Ser, -Lys oder -Arg herleitet, oder der Aminoalkoholrest Z + W ist, der sich von D- oder L-Tyr, -Phe oder -His herleitet, in jedem Fall als solcher oder in Ester- oder Etherform geschützt, z.B. durch eine Schutzgruppe $R^6$, wie sie in Anspruch 8 definiert ist.

15. Polypeptidanaloges nach einem der vorausgehenden Ansprüche, worin G aus folgenden Gruppen ausgewählt ist:

1. 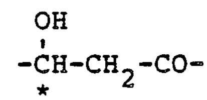 (XXVI)

2. 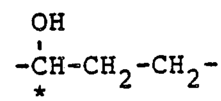 (XXVII)

3. 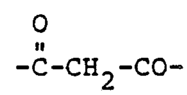 (XXVIII)

4. $-CH_2-CH_2-CO-$ (XXIX)

16. Polypeptidanaloges nach einem der vorausgehenden Ansprüche, bei dem das Polypeptidanaloge in weiter modifizierter Form durch isosterischen Austausch eine oder mehrerer restlicher Peptidbindungen durch

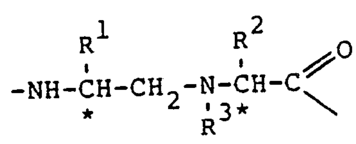 (XIII) "reduzierte" Isosterbindung

oder

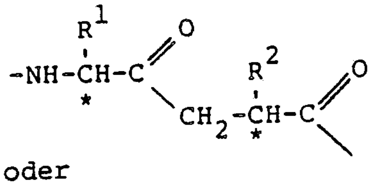 (XIV) "Keto"-Isosterbindung

oder

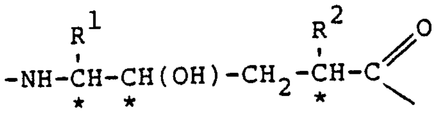 (XV) "Hydroxy"-Isosterbindung

oder

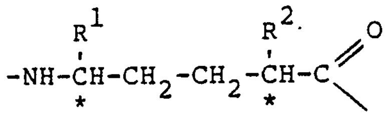 (XVI) "Kohlenwasserstoff"-Isosterbindung

vorliegt, worin die Bedeutung von ★, $R^1$, $R^2$ und $R^3$ die obige ist, wobei insbesondere eine oder beide der Pro-Phe und Phe-His-Bindungen in der Formel V die Stelle des isosterischen Austausches sind.

17. Polypeptidanaloges nach einem der vorausgehenden Ansprüche, bei dem das Polypeptidanaloge in geschützter Form an einer oder mehreren restlichen Amino- oder Amid- (einschließlich Peptid-) Stickstoff-, -Carboxyl-, -Hydroxy- oder anderen reaktiven Gruppen oder in Salzform an Amino-, Imidazol- oder Carboxylgruppen oder als ein Säureadditonssalz an einem oder mehreren basischen Zentren vorliegt.

18. Polypeptidanaloges der allgemeinen Formel:

X-Y-Pro-Phe-His—A—B—His-W (V)

6 7 8 9 10,11 12 13

oder die Teilsequenzen:

X-A-B-His-W (V) (i)

X-His-A-B-His-W (V) (ii)

X-Phe-His-A-B-His-W (V) (iii)

X-Pro-Phe-His-A-B-His-W (V) (iv)

worin:

Pro, Phe und His in substituierter Form vorliegen können, z.B. OH, F, Cl, Br oder Me tragen können;

X = H oder eine Acyl- oder andere N-schützende Gruppe ist, z.B. Acetyl, Pivaloyl, Benzyloxycarbonyl, tert-Butyloxycarbonyl (Boc), Benzoyl oder niedermolekulares Alkyl (hauptsächlich $C_1$—$C_5$) oder ein D- oder L-Aminoacylrest (besonders Pro), der selbst ähnlich N-geschützt sein kann;

Y = D- oder L-His oder in anderer basischer oder aromatischer D- oder L-Aminoacylrest oder nicht vorhanden;

A = 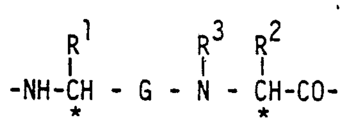 (VI) worin

G = 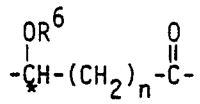 (VII) mit n = 0, 1, 2, 3

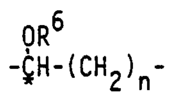 (VIII) n = 1, 2, 3, 4

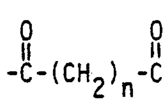 (IX) n = 0, 1, 2, 3

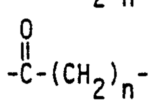 (X) n = 1, 2, 3, 4

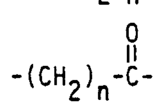 (XI) n = 1, 2, 3

$-(CH_2)_n-$ (XII) n = 3, 4

und worin die Konfiguration an asymmetrischen Zentren ★ entweder R oder S ist und $R^1$ und $R^2$ gleich oder verschieden sind und ${}^iPr$ (Isopropyl), ${}^iBu$ (Isobutyl), Bzl (Benzyl) oder eine andere Aminosäure seitenkette, vorzugsweise eine lipophile oder aromatische, bedeutet;

$R^3$ = H, niedermolekulares Alkyl ($C_1$-$C_5$) oder tert-Butyloxycarbonyl, Benzyloxycarbonyl, ringsubstituiertes Benzyloxycarbonyl, $—SO_2Ph$, $—SO_2C_6H_4CH_3(p)$, Formyl oder eine andere N-Schutzgruppe einschließlich niedermolekularem Acyl ($C_1—C_5$) allgemein;

$R^6$ = H oder niedermolekulares Alkyl, niedermolekulares Acyl, Benzyl, Tetrahydropyranyl oder eine andere Hydroxyl-Schutzgruppe;

B = D- oder L-Val, -Leu oder -Ile oder ein anderer lipophiler D- oder L-Aminoacylrest;

W =

i) —OH als solches oder in geschützter Esterform, z.B. als $—OR^4$, worin $R^4$ = niedermolekulares Alkyl, hauptsächlich $C_1—C_5$-Alkyl und insbesondere ${}^tBu$, oder Cycloalkyl, hauptsächlich $C_3—C_7$-Cycloalkyl oder Bzl, oder eine andere esterbildende Gruppe; oder

ii) $—NH_2$ als solches oder in geschützter Amidform, wie $—NHR^5$ oder $—N(R^5)_2$ (worin $R^5$ = eine N-schützende oder andere Substituentengruppe, z.B. niedermolekulares Alkyl wie für $R^4$ angegeben, und $(R^5)_2$ = zwei solcher Gruppen oder z.B. Cycloalkyl, hauptsächlich $C_3—C_7$-Cycloalkyl) oder als $—NH—(CH_2)_n—Q$ oder $—NR^5—(CH_2)_n—Q$ (worin n = 2 bis 6 und Q = $NH_2$ oder

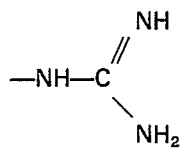

und worin die an Stickstoff gebundenen Wasserstoffatome durch $R^5$ oder $(R^5)_2$ substituiert sein können) oder

iii) ein D- oder L-Serinrest oder D- oder L-Lysin-, -Arginin- oder anderer basischer Aminoacylrest als solcher oder in Amidform, substituierter Amidform oder Esterform, z.B. mit einem Gehalt einer Gruppe oder von Gruppen, wie sie für $R^4$ und $R^5$ oben als gegebenenfalls angegeben sind; oder

iv) ein Aminoalkoholrest, der sich hiervon ableitet, als solcher oder in Ester- oder Etherform geschützt, z.B. mit einem Gehalt einer Gruppe, wie sie oben für $R^4$ angegeben ist, oder

His + W = ein sich von L- oder His- herleitender Alkohol,

wobei ein solches Polypeptid in der obigen Form oder durch isosterischen Austausch einer oder mehrerer restlicher Peptidbindungen beispielsweise durch isosterische reduzierte $—CH_2$-NH—, Keto,

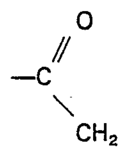

Hydroxy, —CH(OH)$CH_2$— oder $—CH_2—CH_2$-Kohlenwasserstoffbindungen in der Form

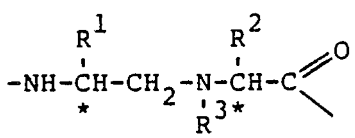

(XIII) "reduzierte" Isosterbindung

oder

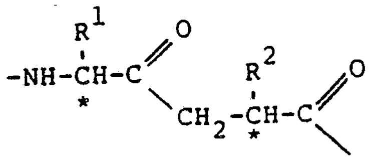

(XIV) "Keto"-Isosterbindung

oder

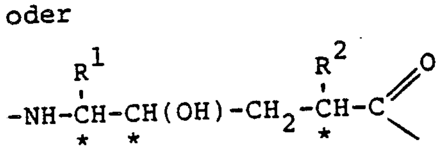

(XV) "Hydroxy"-Isosterbindung

oder

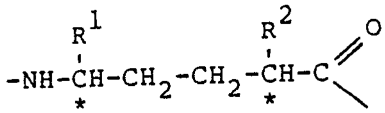 (XVI) "Kohlenwasserstoff"-Isosterbindung

vorliegt, worin die Bedeutung von ★, $R^1$, $R^2$ und $R^3$ die obige ist, und wobei das Polypeptid außerdem in freier Form oder in an einer oder mehreren restlichen Amino- oder Amid-(einschließlich Peptid-)-Stickstoff, Carboxyl-, Hydroxy- oder anderen reaktiven Gruppen geschützter Form oder in Salzform an Amino-, Imidazol- oder Carboxylgruppen vorliegt, insbesondere als physiologisch verträgliche Säureadditionssalze an basischen Zentren.

19. Polypeptidanaloge:

H-His-Pro-Phe-His-Sta-Val-Ile-His-OH

H-Pro-His-Pro-Phe-His-Sta-Val-Ile-His-Lys-OH

oder entsprechende Analoge, worin die Bindung —$CH(OH)CH_2CO$— des Statinrestes durch eine der in Anspruch 5 angegebenen Bindungen ausgetauscht ist.

20. Polypeptidanaloges nach einem der vorausgehenden Ansprüche für die Verwendung in einem diagnostischen Test für hohe Reninspiegel, bei dem ein Polypeptidanaloges verabreicht wird, wobei ein positives Ergebnis durch einen Abfall des Blutdruckes in Abhängigkeit von dieser Verabreichung angezeigt wird, oder für die Verwendung bei der Behandlung von Herzfehleren oder erhöhtem Blutdruck, bei der eine wirksame Menge eines Polypeptidanalogen verabreicht wird.

21. Arzneimittel mit einem Polypeptidanalogen nach einem der verausgehenden Ansprüche 1 bis 19 in einem pharmazeutisch verträglichen Medium.

**Revendications**

1. Analogues de polypeptides de formule générale:

X-Y-Pro-Phe-His-A-B-His-W (V)

ou les séquences partielles:

X-A-B-His-W (V) (i)

X-His-A-B-His-W (V) (ii)

X-Phe-His-A-B-His-W (V) (iii)

X-Pro-Phe-His-A-B-His-W (V) (iv)

dans lesquelles:

X représente un atome d'hydrogène, un groupe protecteur de l'azote de fonction amino ou un reste amino acyle;

Y représente un reste D- ou L- amino acyle basique ou aromatique;

A représente 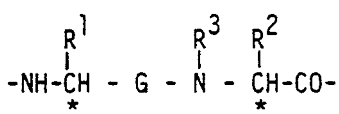 (VI) ou

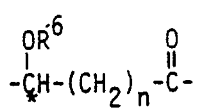 (VII) avec n = 0, 1, 2, 3

G représente 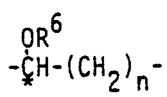 (VIII) n = 1, 2, 3, 4

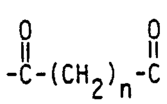 (IX) n = 0, 1, 2, 3

$$-\overset{O}{\overset{\|}{C}}-(CH_2)_n- \qquad (X) \qquad n = 1, 2, 3, 4$$

$$-(CH_2)_n-\overset{O}{\overset{\|}{C}}- \qquad (XI) \qquad n = 1, 2, 3$$

$$-(CH_2)_n- \qquad (XII) \qquad n = 3, 4$$

et la configuration des centres d'asymetrie ★ est R ou S, et $R^1$ et $R^2$ identiques ou différents, représentent des chaînes latérales d'amino acides;

$R^3$ représente un atome d'hydrogène ou un groupe protecteur de l'azote de fonction amino;

$R^6$ représente un atome d'hydrogène ou un groupe protecteur de fonction hydroxyle;

B est un reste amino acyle lipophile; et

W représente un groupe hydroxyle, un groupe amide azoté, ou un reste amino acyle basique ou un reste amino alcool qui en dérive; ou His+ W représente le rest amino alcool correspondant à His.

2. Analogues de polypeptide selon la revendication 1, dans lesquels:

$$G = -\overset{OR^6}{\overset{|}{\underset{\star}{C}}}H-CH_2-\overset{O}{\overset{\|}{C}}- \qquad (VII)$$

$$-\overset{OR^6}{\overset{|}{\underset{\star}{C}}}H-(CH_2)_2- \qquad (VIII)$$

$$-\overset{O}{\overset{\|}{C}}-CH_2-\overset{O}{\overset{\|}{C}}- \qquad (IX)$$

$$-\overset{O}{\overset{\|}{C}}-(CH_2)_2- \qquad (X)$$

$$-(CH_2)_2-\overset{O}{\overset{\|}{C}}- \qquad (XI)$$

$$-(CH_2)_3- \qquad (XII)$$

3. Analogue de polypeptide selon la revendication 1, dans lequel un ou plusieurs desdits groupes Pro Phe et His portent des substituants sur le noyau, Pro portant OH comme substituant, Phe portant OH, F, Cl, Br ou Me comme substituants, His portant Me comme substituant, ou bien ils sont remplacés, respectivément, par un groupe 4-hydroxyproline (HPro) ou pGlu; Tyr, Phe (4-Cl) ou (4-F); His (Me) ou spinacine.

4. Analogue de polypeptide selon la revendication 1 ou 2, dans lequel ledit groupe protecteur X est un groupe acyle ou alkyle inférieur et, en particulier, ledit groupe acyle est un groupe acétyle, pivaloyle, t-butoxycarbonyle (Boc), benzyloxycarbonyle ou benzoyle, et ledit groupe alkyle inférieur est un groupe alkyle en $C_1$ à $C_5$.

5. Analogue de polypeptide selon la revendication 1 ou 2, dans lequel ledit groupe amino acyle X est un groupe D- ou L- Pro, Val ou Ile, ou Gly, et/ou est sous forme protégée portant un groupe tel qu'indiqué à la revendication 3.

6. Analogue de polypeptide selon l'une quelconque des revendications précédentes, dans lequel ledit reste amino acyle Y est un reste D- ou L-His.

7. Analogue de polypeptide selon l'une quelconque des revendications précédentes, dans lequel lesdites chaînes latérales d'amino acides $R^1$ et $R^2$ sont lipophiles ou aromatiques, et représentent par exemple un groupe isopropyle, isobutyle et benzyle, et en particulier représentent les chaînes Leu Ile Val Phe cyclohexyl-Ala adamantyl-Ala ou Phe Tyr Trp ou His.

8. Analogue de polypeptide selon l'une quelconque des revendications précédentes, dans lequel ledit

groupe protecteur $R^3$ est un groupe alkyle inférieur, acyle inférieur, $-SO_2Ph$, $-SO_2C_6H_4CH_3(p)$, t-butoxy carbonyle (Boc), benzyloxycarbonyle ou benzyloxycarbonyle substitué sur le noyau, et en particulier ledit groupe alkyle ou acyle est un groupe en $C_1$ à $C_5$.

9. Analogue de polypeptide selon l'une quelconque des revendications précédentes, dans lequel ledit groupe $R^6$ protecteur d'une fonction hydroxyle est un groupe alkyle inférieur ou acyle inférieur, en particulier un groupe alkyle ou acyle en $C_1$ à $C_5$, ou un groupe benzyle ou tetrahydropyrannyle.

10. Analogue de polypeptide selon l'une quelconque des revendications précédentes, dans lequel ledit reste B est un reste D- ou L-Val ou Ile.

11. Analogue de polypeptide selon l'une quelconque des revendications précédentes, dans lequel groupe hydroxyle W est protégé sous forme d'ester, par exemple par un groupe alkyle inférieur ou cycloalkyle, ou Bzl, et en particulier dans lequel ledit groupe alkyle inférieur est un groupe alkyle en $C_1$ à $C_5$ et ledit groupe cycloalkyle est un groupe cycloalkyle en $C_3$ à $C_7$.

12. Analogue de polypeptide selon l'une quelconque des revendications précédentes, dans lequel ledit groupe amide azoté W est sous forme protégée, en particulier il est

i) un groupe $-NHR^5$ ou $-N(R^5)_2$ dans lequel $R^5$ représente un groupe alkyle, inférieur et $(R^5)_2$ représente deux groupes alkyle inférieurs identiques ou différents, ou un groupe cycloalkyle, et, en particulier, le ou chaque groupe alkyle est un groupe alkyle en $C_1$ à $C_5$ et ledit groupe cycloalkyle est un groupe cycloalkyle en $C_3$ à $C_7$, ou

ii) un groupe $-NH-(CH_2)_n-Q$ ou $NR^5-(CH_2)_n-Q$ où n vaut 2 à 6 et Q représente un groupe amino ou guanidyle, $R^5$ est un groupe alkyle inférieur, et en particulier un ou plusieurs des atomes d'hydrogène fixés sur l'azote dans ledit groupe protecteur est ou sont remplacés par $R^5$ ou $(R^5)_2$, répondant à la définition ci-dessus.

13. Analogue de polypeptide selon l'une quelconque des revendications précédentes, dans lequel ledit reste amino acide W est un reste D- ou L- Ser, Lys ou Arg, tel quel ou sous forme:

i) d'un amide ou d'un amide protégé, en particulier lorsqu'il est protégé par un groupe tel qu'indique à la revendication 12, ou

ii) d'un ester, en particulier quand le groupe estérifiant est tel qu'indiqué à la revendication 11.

14. Analogue de polypeptide selon l'une quelconque des revendications précédentes, dans lequel ledit reste amino alcool W est le reste amino alcool provenant de D- ou L- Ser, Lys ou Arg, ou bien ledit reste amino alcool Z + W dérive de D- ou L- Tyr Phe ou His, dans l'un ou l'autre cas sous forme telle quelle ou sous forme protégée d'ester ou d'éther, par exemple protégée par un groupe protecteur $R^6$ tel qu'indiqué à la revendication 8.

15. Analogue de polypeptide selon l'une quelconque des revendications précédentes, dans lequel G est choisi parmi:

1. 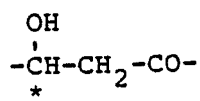 (XXVI)

2. 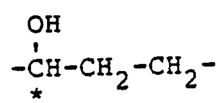 (XXVII)

3. 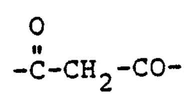 (XXVIII)

4. $-CH_2-CH_2-CO-$ (XXIX)

16. Analogue de polypeptide selon l'une quelconque des revendications précédentes, dans lequel ledit analogue de polypeptide est sous forme encore modifiée par remplacement isostère d'une ou plusieurs liaisons peptidiques restantes par:

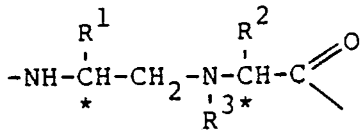

(XIII) liaison isostère ''réduite''

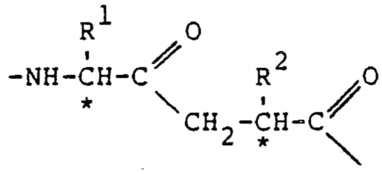

(XIV) liaison isostère ''ceto''

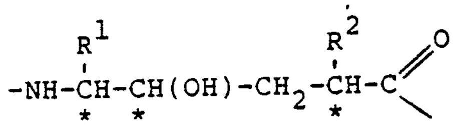

(XV) Liaison isostère ''hydroxy''

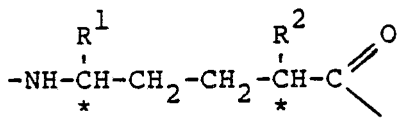

(XVI) Liaison isostère ''hydrocarbure''

la signification de ★, $R^1$, $R^2$ et $R^3$ étant comme ci-dessus, en particulier analogue dans lequel une des liaisons Pro-Phe et Phe-His, ou les deux, est ou sont, dans la formule 5, le site du remplacement isostère.

17. Analogue de polypeptide selon l'une quelconque des revendications précédentes, dans lequel ledit analogue de polypeptide est sous forme protégée sur un ou plusieurs groupes azotés amino ou amide (ce qui comprend un peptide), carboxyle, hydroxy ou un autre groupe réactif, ou sous forme de sel au niveau des groupes amino, imidazole ou carboxyle, ou est sous forme d'une sel d'addition avec un acide sur un ou plusieurs centre basiques.

18. Analogue de polypeptide de formule générale:

X-Y-Pro-Phe-His—A—B—His-W (V)

6 7 8 9 10,11 12 13

ou les séquences partielles:

X-A-B-His-W (V) (i)

X-His-A-B-His-W (V) (ii)

X-Phe-His-A-B-His-W (V) (iii)

X-Pro-Phe-His-A-B-His-W (V) (iv)

formules dans lesquelles:

Pro, Phe, et His peuvent être sous forme substitué, et peuvent porter par example OH, F, Cl, Br, ou Me;

X représente un atome d'hydrogène ou un groupe de protection d'azote, acylé ou autre groupe protecteur, par exemple un groupe acétyle, pivaloyle, benzyloxycarbonyle, t-butyloxycarbonyle (Boc), benzoyle ou alkyle inférieur (principalement en $C_1$ à $C_5$); ou un reste D- ou L- amino acyle (en particulier Pro), qui peut lui même être protégé de façon analogue sur l'azote;

Y représente D- ou L-His ou un autre reste D- ou L- amino acyle basique ou aromatique, ou est absent;

A représente 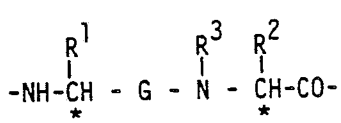 (VI) ou

G représente 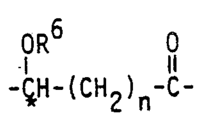 (VII) avec n = 0, 1, 2, 3

$-\underset{*}{C}H(OR^6)-(CH_2)_n-$ (VIII) n = 1, 2, 3, 4

$-\overset{O}{\overset{\|}{C}}-(CH_2)_n-\overset{O}{\overset{\|}{C}}$ (IX) n = 0, 1, 2, 3

$-\overset{O}{\overset{\|}{C}}-(CH_2)_n-$ (X) n = 1, 2, 3, 4.

$-(CH_2)_n-\overset{O}{\overset{\|}{C}}-$ (XI) n = 1, 2, 3

$-(CH_2)_n-$ (XII) n = 3, 4

et la configuration des centres d'asymétric ★ est R ou S, $R^1$ et $R^2$, identiques ou différents, représentant un groupe $^{i}Pr$ (isopropyle), $^{i}Bu$ (isobutyle), Bzl (benzyle) ou une autre chaîne latérale amino acide, de préférence lipophile ou aromatique;

$R^3$ représente H; un groupe alkyle inférieur ($C^1$ à $C_5$) ou un groupe t-butyloxycarbonyle, benzyloxycarbonyle, benzyloxycarbonyle substitué sur le noyau, $—SO_2PH$, $—SO_2C_6H_4CH_3(p)$, formyle ou un autre groupe protecteur de l'azote, ce qui comprend de façon générale un groupe acyle inférieur ($C_1$ à $C_5$);

$R^6$ représente un atome d'hydrogène ou un groupe alkyle inférieur, acyle inférieur, benzyle, tétrahydropyrannyle, ou un autre groupe protecteur de fonction hydroxyle;

B représente D- ou L-Val, Leu ou Ile ou un autre reste D- ou L- amino acyle lipophile;

et W représente:

i) —OH tel quel ou protégé sous forme d'ester, par exemple sous forme de $—OR^4$, où $R^4$ représente un groupe alkyle inférieur, principalement en $C_1$ à $C_5$ et en particulier $^{t}Bu$, ou un groupe cycloakyle, en particulier en $C_3$ à $C_7$, ou Bzl, ou un autre groupe formateur d'ester; ou

ii) $—NH_2$, tel quel ou sous forme protégée comme amide $—NHR^5$ ou $—N(R^5)_2$ (où $R^5$ représente un groupe protecteur de l'azote ou un autre groupe substituant par exemple un groupe alkyle inférieur comme dans le cas de $R^4$ et $(R^5)_2$ qui représentent deux groupes de ce genre ou par exemple un groupe cycloalkyle, principalement en $C_3$ à $C_7$), ou sous forme de $—NH—(CH_2)_n—Q$ ou $—NR^5—(CH_2)_n—Q$ (où n vaut 2 à 6, et Q représente $NH_2$ ou

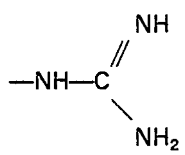

et l'un quelconque des atomes d'hydrogène fixés sur l'azote peut être remplacé par $R^5$ ou $(R^5)_2$); ou

iii) un reste D- ou L- sérine ou D- ou L- lysine, arginine ou un autre reste amino acyle basique, tel quel, ou sous forme d'amide, sous forme d'amide substitué ou sous forme d'ester, par exemple contenant un ou des groupes correspondant à ce qui est indiqué pour $R^4$ et $R^5$ ci-dessus selon le cas; ou

iv) un reste amino alcool qui en provient, tel quel ou protégé sous forme d'ester ou d'éther contenant par exemple un groupe tel qu'indiqué pour $R_4$ ci-dessus, ou His + W représente un alcool provenant de L- ou D-His, un tel polypeptide étant sous la forme ci-dessus ou sous forme modifiée par remplacement isostère d'une ou plusieures liaisons peptidiques restantes, par exemple par des liaisons isostères $—CH_2—NH—$ "réduite", cétonique,

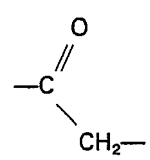

hydroxy, $—CH(OH)—CH_2—$, ou une liaison isostère $—CH_2—CH_2—$ d'hydrocarbure sous la forme:

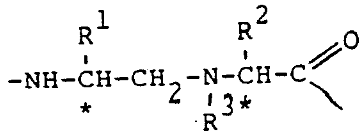

(XIII) liaison isostère ''réduite''

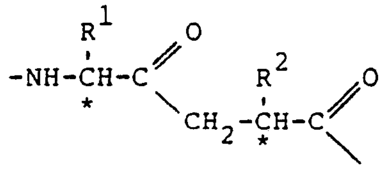

(XIV) liaison isostère ''ceto''

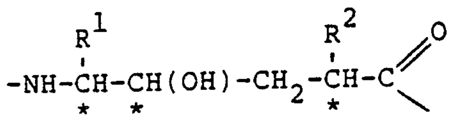

(XV) Liaison isostère ''hydroxy''

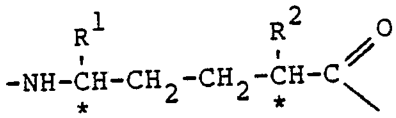

(XVI) Liaison isostère ''hydrocarbure''

le sens de ★, $R^1$, $R^2$, et $R^3$ étant comme défini ci-dessus et ledit polypeptide étant en outre sous forme libre ou sous forme protégée sur un ou plusieurs groupes azotés amino ou amide (y compris peptide), carboxyle, hydroxy ou autres groupes réactifs restants, ou sous forme de sel au niveau des groupes amino, imidazole ou carboxyle, en particulier sous la forme de leurs sels d'addition avec des acides physiologiquement acceptables, au niveau des centres basiques.

19. Les analogues de polypeptide:

H-His-Pro-Pre-His-Sta-Val-Ile-His-OH

H-Pro-His-Pro-Phe-His-Sta-Val-Ile-His-Lys-OH

ou les analogues correspondants, dans lesquels la liaison —$CH(OH)CH_2CO$— du reste statine est remplacée par une des liaisons indiquées à la revendication 15.

20. Analogue de polypeptide selon l'une quelconque des revendications précédentes, destiné à servir dans un essai de diagnostic d'un état à taux élevé de rénine, essai dans lequel on administre un analogue de polypeptide, un résultant positif étant indiqué par un chute de la pression sanguine liée à ladite administration, ou analogue destiné à servir au traitement d'un défaillance cardiaque ou d'une hypertension, cas dans lequel on administre une quantité efficace d'un analogue de polypeptide.

21. Composition pharmaceutique comprenant un analogue de polypeptide selon l'une quelconque des revendications 1 à 19 précédentes, dans un milieu pharmaceutiquement acceptable.